(19) Europäisches Patentamt / European Patent Office / Office européen des brevets

(11) **EP 4 656 183 A1**

(12) # EUROPEAN PATENT APPLICATION

(43) Date of publication:
**03.12.2025 Bulletin 2025/49**

(21) Application number: **25382104.5**

(22) Date of filing: **07.02.2025**

(51) International Patent Classification (IPC):
**A61K 9/00** *(2006.01)*    **A61K 9/08** *(2006.01)*
**A61K 9/19** *(2006.01)*    **A61K 38/26** *(2006.01)*
*A61K 47/02* *(2006.01)*    *A61K 47/18* *(2017.01)*
*A61K 47/26* *(2006.01)*

(52) Cooperative Patent Classification (CPC):
**A61K 9/0019; A61K 9/08; A61K 38/26;**
A61K 47/02; A61K 47/183; A61K 47/26

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 31.05.2024  EP 24382587
             14.01.2025  US 202519019721
             04.02.2025  EP 25155776

(71) Applicant: **Galenicum Health SLU**
     **08950 Esplugues de Llobregat (ES)**

(72) Inventors:
• **OSUNA LÓPEZ, JAIME**
  **08950 EPSLUGUES DE LLOBREGAT (ES)**
• **GÓMEZ COELLO, LUIS**
  **08950 ESPLUGUES DE LLOBREGAT (ES)**

(74) Representative: **Galenicum Health S.L.U.**
     **CL Sant Gabriel n°50**
     **08950 Esplugues de Llobregat (ES)**

(54) **TIRZEPATIDE COMPOSITIONS AND PREPARATION METHOD**

(57)    The present invention relates to stable pharmaceutical compositions comprising tirzepatide, a dual Glucose-Dependent Insulinotropic Polypeptide (GIP) and Glucagon-Like Peptide-1 (GLP-1) receptor agonist. In particular, the invention provides aqueous formulations of tirzepatide containing non-inorganic buffering agents that improve chemical stability, bioavailability, and patient tolerability. The invention further encompasses pharmaceutical compositions formulated for enhanced storage stability and reduced aggregation, ensuring optimal therapeutic efficacy in the treatment of diabetes mellitus and obesity.

EP 4 656 183 A1

**Description**

The present invention relates to stable pharmaceutical compositions comprising tirzepatide, a dual Glucose-Dependent Insulinotropic Polypeptide (GIP) and Glucagon-Like Peptide-1 (GLP-1) receptor agonist. In particular, the invention provides aqueous formulations of tirzepatide containing non-inorganic buffering agents that improve chemical stability, bioavailability, and patient tolerability. The invention further encompasses pharmaceutical compositions formulated for enhanced storage stability and reduced aggregation, ensuring optimal therapeutic efficacy in the treatment of diabetes mellitus and obesity.

**BACKGROUND**

The development of pharmaceutical compositions comprising therapeutic peptides such as tirzepatide is of significant interest due to their potential in treating conditions like diabetes and obesity. Tirzepatide, as a dual GIP/GLP-1 receptor agonist, offers improved glycemic control and weight reduction benefits over traditional GLP-1 analogues. However, like other peptide-based pharmaceuticals, tirzepatide is susceptible to degradation, requiring careful formulation to ensure stability, efficacy, and safety. These peptides are prone to chemical instability, such as oxidation, hydrolysis, and deamidation, which may affect their structural integrity and therapeutic function. Additionally, physical instability, such as aggregation or precipitation, may lead to reduced bioavailability and potential immunogenic responses. Developing stable tirzepatide formulations is, therefore, a key challenge in pharmaceutical sciences.

Inorganic buffers such as dibasic sodium phosphate are used in commercial formulations of tirzepatide. EP3810201 and WO2024006662 disclose pharmaceutical compositions of tirzepatide comprising sodium phosphate dibasic hepathydrate as a buffering agent.

While inorganic buffers like sodium phosphate offer effective pH control, they may also present certain disadvantages in pharmaceutical formulations. These include potential incompatibility with biologically active peptides, leading to precipitation, hydrolysis, or unwanted interactions that reduce drug stability. Additionally, sodium phosphate buffers can exacerbate injection site irritation in some patients due to their ionic nature. Another challenge is their pH-dependent solubility, which can lead to crystallization under certain storage conditions, thereby compromising the formulation's stability and efficacy. Furthermore, phosphate buffers have been associated with calcium chelation, potentially leading to precipitate formation in the presence of divalent cations. Given these drawbacks, alternative buffering agents are desirable for improving the stability, safety, and tolerability of tirzepatide formulations.

Another challenge is the avoidance of fibril formation, a process by which these peptides tend to form well-ordered, thread-like macromolecular aggregates, poses a significant challenge in GLP-1 analogues formulation. In the literature, heat treatment of GLP-1 solutions has been suggested for increasing the shelf life and stability of the pharmaceutical solutions due to fibril formation.

Fibrils in parenteral compositions present several significant disadvantages that impact the safety, efficacy, and regulatory compliance of pharmaceutical products. Immunogenicity is a major concern, as fibrils can provoke an immune response, potentially leading to adverse reactions in patients, ranging from mild allergic responses to severe immunological complications. The presence of fibrils also signifies a loss of therapeutic efficacy, as aggregated proteins are often denatured and incapable of performing their intended biological functions. This aggregation indicates instability, compromising the product's shelf-life and necessitating stringent storage conditions. Additionally, the physical presence of fibrils can pose safety risks, such as causing blockages in blood vessels or tissues, which could lead to serious health issues like embolism or inflammation. Hence, minimizing fibril formation is crucial for ensuring the overall integrity and success of parenteral pharmaceutical applications.

According to WO2006051110A2, fibrillation in GLP-1 analogues peptides solutions can be reduced by heating the solution of said peptides between 50° C and 95 °C, at a pH between 8.0 to 10.5 and then continue the heating for between 3 minutes and 180 minutes. This method would allow the fibrils to dissolve in their initial state and delay its formation. However, heating a pharmaceutical reactor to reach these temperatures requires a significant amount of energy, thereby increasing manufacturing costs and leading to higher emissions of greenhouse gases.

WO2020127476A1 discloses a method for the preparation of pharmaceutical solution comprising a GLP-1 peptide analogue which involves heating the solution to a temperature of 26-49°C. However, heating a pharmaceutical reactor to reach these temperatures requires a significant amount of energy, thereby increasing manufacturing costs and leading to higher emissions of greenhouse gases.

WO202061310 discloses GLP-1 and GIP dual receptor agonists compositions, wherein the buffer is inorganic (e.g. hydrogen phosphate) or alternatively the buffer is a citrate buffer. One potential drawback of citrate buffer is its pH range limitation. Citrate has three pKa values (3.1, 4.8, and 6.4), making it most effective as a buffer in the acidic to mildly neutral range (pH 3-6.5). That said, commercial tirzepatide formulations are in the pH range of 7.0-7.5, the reason why it is not optimal to use it a generic tirzepatide pharmaceutical formulation.

Commercial trizepatide compositions need to be stored under refrigerated conditions (i.e. 2ºC-8ºC), and are only stable at room temperature for 21 days.

Therefore, there is an unresolved need to formulate GLP-1 and GIP dual receptor agonists compositions with improved stability. The present invention is directed towards addressing the shortcomings and challenges outlined above.

## SUMMARY OF THE INVENTION

The present invention provides a pharmaceutical composition comprising a GLP-1 and GIP dual receptor agonist wherein the buffer is said pharmaceutical composition is suitable to maintain a pH between 6.5 to 8.5 throughout the shelf life of the product while avoiding excessive degradation of the said dual receptor agonist.

Inorganic buffers (e.g. sodium phosphate dibasic) are used in commercial products comprising GLP-1 receptor agonists such as liraglutide (Victoza®), semaglutide (Ozempic®, Wegovy®) and also in those commercial products comprising GLP-1 and GIP dual receptor agonists, such as tirzepatide (Mounjaro®, Zepbound®).

Inorganic buffers are widely used in pharmaceutical compositions comprising the mentioned agonists due to its well-established safety profile and relatively low interaction with the active ingredients. However, the inventors have surprisingly found that stable pharmaceutical formulations according to the invention comprising a GLP-1 and GIP dual receptor agonist prepared with a buffer other than an inorganic buffer show improved stability.

In addition, the present invention aims to provide a method of manufacturing of the pharmaceutical compositions of the invention which is more energy efficient and confers a lower impurity formation to the manufactured product.

Each of the aspects and embodiments of the present invention described herein may be combined with one or more aspects and embodiments of the present invention.

In a first aspect, the invention concerns a pharmaceutical composition comprising a GLP-1 and GIP dual receptor agonist and a non-inorganic buffer having a pKa of at least 6.6 or above.

The pharmaceutical compositions according to the invention have lower impurity content over time and less individual and total impurities than pharmaceutical solutions comprising the mentioned dual receptor agonists and inorganic buffers.

A second aspect of the present invention relates to a method for the preparation of the pharmaceutical composition of the first aspect comprises the following steps:

   i) preparing a solution comprising the tonicity modifier, the buffering agent, the GLP-1 and GIP dual receptor agonist and water for injection; and
   ii) adjusting the pH of the final solution between 6.5-8.5, preferably between 6.5-7.5, with a pH adjuster.

In a third aspect, a cartridge comprising the pharmaceutical composition of the first aspect or the pharmaceutical composition prepared according the second aspect is disclosed.

## DETAILED DESCRIPTION OF THE INVENTION

As used in the present disclosure, the following words, phrases, and symbols are generally intended to have the meanings as set forth below, except to the extent that the context in which they are used indicates otherwise.

All percentages are expressed by weight (w/w) and as used herein are referred to the total weight of the composition, unless specifically noted otherwise.

The term "active ingredient" as used herein refers to a therapeutically active compound, as well as any pharmaceutically acceptable hydrates and solvates of the compound.

The term "buffer" as used herein refers to a chemical compound in a pharmaceutical composition that reduces the tendency of pH of the composition to change over time as would otherwise occur due to chemical reactions.

The term "inorganic buffer" as used herein refers to a buffering agent in a pharmaceutical composition that is derived from an inorganic acid or its conjugate base and functions to maintain a stable pH by resisting changes due to acid-base reactions. Non-limiting examples of inorganic buffers include phosphate buffers (e.g., sodium phosphate, potassium phosphate), carbonate buffers (e.g., sodium bicarbonate, potassium bicarbonate), and borate buffers (e.g., sodium borate, boric acid).

The term "non-inorganic buffer" as used herein refers to any buffering agent that is not encompassed within the definition of an inorganic buffer. Non-inorganic buffers include, but are not limited to, organic acid-base buffers (e.g., citrate, acetate, lactate), zwitterionic buffers (e.g., HEPES, MOPS, PIPES), and amine-based buffers (e.g., Tris, BES, TES). These buffers function to maintain pH stability in pharmaceutical compositions by resisting changes due to acid-base reactions.

The term "organic acid-base buffer" as used herein refers to a non-inorganic buffering agent derived from an organic acid and its conjugate base, or an organic base and its conjugate acid. These buffers function by reversible protonation and deprotonation to resist pH fluctuations, typically in the acidic to mildly neutral pH range.

The term "organic amine-based buffer" as used herein refers to a non-inorganic buffering agent that contains one or more amine ($-NH_2$) functional groups and is derived from an organic compound. These buffers function to resist pH fluctuations by reversible protonation and deprotonation of the amine group, typically in the neutral to alkaline pH range. Non-limiting examples of organic amine-based buffers include Tris (tris(hydroxymethyl)aminomethane), Bicine (N,N-bis(2-hydroxyethyl)glycine), TES (N-tris(hydroxymethyl)methyl-2-aminoethanesulfonic acid), TAPS (N-tris(hydroxymethyl)methyl-3-aminopropanesulfonic acid), Tricine (N-tris(hydroxymethyl)methylglycine), and Bis-Tris (2,2-bis(hydroxymethyl)-2,2',2"-nitrilotriethanol).

The term "zwitterionic buffer" as used herein refers to a non-inorganic buffering agent that contains both a positively charged (cationic) and a negatively charged (anionic) functional group within the same molecule at a given pH, allowing it to resist pH changes effectively while maintaining overall electrical neutrality. Zwitterionic buffers are particularly useful in biological and pharmaceutical compositions due to their minimal interaction with biomolecules and metal ions. Non-limiting examples of zwitterionic buffers include Good's buffers such as HEPES (pKa ~7.5), MOPS (pKa ~7.2), MES (pKa ~6.1), and PIPES (pKa ~6.8).

The term "pH adjuster" as used herein refers to pharmaceutically acceptable excipients which are added to the solution of the active agent to adjust the pH to a certain value. Such pH adjusters can be alkaline or acid agents and may comprise inorganic salts as well as organic acids or salts of organic acids. Examples of pH adjusters are HCl or NaOH.

The term "pharmaceutically acceptable" as used herein indicates that the substance or composition must be compatible chemically and/or toxicologically, with the other ingredients in the composition, and/or the mammal being treated therewith.

The term "pharmaceutical composition" as used herein means a product comprising an active compound or a salt thereof together with pharmaceutical excipients such as buffer, preservative and tonicity modifier, said pharmaceutical composition being useful for treating, preventing or reducing the severity of a disease or disorder by administration of said pharmaceutical composition to a person. Thus, a pharmaceutical composition is also known in the art as a pharmaceutical formulation.

The term "pKa" as used herein refers to the negative base-10 logarithm of the acid dissociation constant (Ka) of a compound, representing the pH at which the compound exists in equilibrium between its protonated and deprotonated forms. The pKa value of a buffer determines its optimal pH range for resisting pH fluctuations in a pharmaceutical composition. Lower pKa values correspond to stronger acids, while higher pKa values indicate weaker acids. Non-limiting examples include the pKa of citric acid (~3.1, 4.8, 6.4), phosphate buffer (~7.2), HEPES (~7.5), and Tris (~8.1).

The term "preservative" as used herein refers to a chemical compound which is added to a pharmaceutical composition to prevent or delay microbial activity (growth and metabolism). Examples of pharmaceutically acceptable preservatives are phenol, m-cresol, methyl p-hydroxybenzoate, propyl p-hydroxybenzoate, benzoic acid, benzyl alcohol, benzyl benzoate, 2-phenoxyethanol, butyl p-hydroxybenzoate, 2-phenylethanol, benzyl alcohol, chlorobutanol, acetone sodium bisulfite, benzalkonium chloride, benzethonium chloride and thiomerosal.

The term "stable" as used herein refers to any pharmaceutical composition comprising the active ingredient having a sufficient physical and chemical stability to allow storage under any of the general storage conditions as defined by ICH Q1A (R2).

The term "tonicity modifier" as used refers to a chemical compound in a pharmaceutical composition that serves to modify the osmotic pressure of the pharmaceutical composition so that the osmotic pressure becomes closer to that of human plasma. The tonicity modifier is also known in the art as "isotonicity agent". Isotonicity agents include mannitol, sorbitol, lactose, propylene glycol dextrose, trehalose, sodium chloride, potassium chloride, glycerol, glycerin, etc.

The quantitative determination of tirzepatide by UPLC analysis was carried out using ACQUITY UPLC H-Class PLUS system, Aeris Peptide XB C18 100Å 4.6 mm x 250 mm, 3.6 μm column was used. A Segurity Guard ULTRA cartridge, UHPLC C18-Peptide 4.6 mm ID pre-column was used. The apparatus was equipped with a manual injector and UV detector. The injection valve was a Rheodyne with a capacity of 20 μl. Mobile phase A (0.5% TFA in Water: Methanol (95:5, v/v%)) and Mobile phase B (0.5% TFA in Acetonitrile:Methanol:Water (90:5:5, v/v/v%) with 6.0 mL NH3 30%) were used at a ratio 40:60. As a diluent, 0,025% v/v Ammonia in water was used. The mobile phases were filtered through a 0.45 μm membrane filter and sonicated before use. It was pumped through the column at a flow rate of 0.8 ml/min. Injection volume was 5 μl and the column was maintained at 55ºC. The detection was monitored at 220 nm and the run time was set as 35 minutes. The amount of tirzepatide in the samples was determined by comparison with appropriate external standard curves obtained applying the least square linear regression analysis.

The quantitative determination of tirzepatide impurities by UPLC was carried out using an ACQUITY UPLC H-Class PLUS system, Acquity UPLC Peptide CSH C18 130Å 2.1 x 150mm, 1.7μm (Two columns connected in series with column coupler) column was used. An Acquity UPLC Peptide CSH C18 Vanguard Pre-Column 130Å 5 x 2.1mm, 1.7μm pre-column was used. Mobile phase A (Buffer solution 4mM: Methanol: TFA (950:50:1, v/v/v%) adjusted to pH 7.00 with Ammonia solution) and Mobile phase B (Acetonitrile: Methanol: Water: TFA (500:450:50:1, v/v/v/v%)) were used at a ratio 26:74. As a diluent, 0,025% v/v Ammonia in water was used. The mobile phases were filtered through a 0.45 μm membrane filter and sonicated before use. It was pumped through the column at a flow rate of 0.2 ml/min. Injection volume

was 4 μl and the column was maintained at 55ºC. The detection was monitored at 215 nm and the run time was set as 145 minutes. The percentage of other impurities are calculated by the following equation:

$$\% \text{ Impurity} = (As \times Wst \times P \times fds \times Ds \times Cf)/(Ast \times Ws \times fdst \times C)$$

Where,

As: Impurity peak area in test solution
Ast: Tirzepatide peak area in reference solution
Wst: Weight of tirzepatide standard in Reference Solution (mg)
Ws: Weight of sample in test solution (g)
P: Purity of tirzepatide standard as is (%), into account KF determination before use (H)

$$P = P \text{ tirzepatide} \times (100 - (H))/100$$

fdst: Dilution factor of Reference Solution (2500)
fdS: Dilution factor of test solution (10)
Ds: Density of product (g/mL)
C: Content of tirzepatide in 1 mL, in mg (6.0)
Cf: Correction factor (1 / RRF = Tirzepatide RF /Impurity RF)

The term "unknown impurity" as used herein refers to an impurity of unknown structure having a specific relative retention time (RRT or $t_{Rr}$) in each case. The percentage of each impurity is calculated as explained above from the results of the analysis under the UPLC conditions set forth above.

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide.

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, PEGylations and the like.

The term "dual GLP-1 and GIP agonist", as used herein, refers to a class of drugs that activate both the glucagon-like peptide-1 (GLP-1) receptor and the glucose-dependent insulinotropic polypeptide (GIP) receptor. These agents mimic the actions of the endogenous incretin hormones GLP-1 and GIP, which are released by the gut in response to food intake, thereby enhancing insulin secretion, reducing blood glucose levels, and regulating energy balance. Non-limiting examples of dual GLP-1 and GIP agonists known in the art include tirzepatide, CT-388, VK2735, CT-868, and HRS9531.

All percentages, parts and ratios herein used are by weight unless specifically noted otherwise. As used herein, the term "about" refers preferably to a range that is ±10%, preferably ±5%, or more preferably ±1% of a value with which the term is associated.

Unless otherwise indicated, all the analysis methods are carried out according to the European Pharmacopoeia 10th edition.

The inventors have found out that a pharmaceutical composition according to the invention has a low level of impurities.

The first aspect of the invention relates to a pharmaceutical composition comprising a GLP-1 and GIP dual receptor agonist and a non-inorganic buffer having a pKa of at least 6.6 or above.

In an embodiment, the non-inorganic buffer is a zwitterionic buffer. In a preferred embodiment, the zwitterionic buffer is HEPES, MOPS, MES, and PIPES. In an even more preferred embodiment, the zwitterionic buffer is HEPES.

In another embodiment, the non-inorganic buffer is an organic amine-based buffer. In a preferred embodiment, the organic amine-based buffer is Tris, Bicine, TES, TAPS, Tricine, and Bis-Tris. In an even more preferred embodiment, the organic amine-based buffer is Tris.

In another embodiment, the non-inorganic buffer is an organic acid-base buffer.

In another embodiment, the GLP-1 and GIP dual receptor agonist is tirzepatide.

In another embodiment, the pharmaceutical composition is free from an inorganic buffer.

In another embodiment, the pharmaceutical composition is explicitly free from a phosphate buffer, non limitng examples of phsophate buffers comprise sodium phosphate buffer, potassium phosphate buffer, phosphate-buffered saline (PBS), Sørensen's phosphate buffer, McIlvaine phosphate-citrate buffer and phosphate-carbonate buffer. In a more preferred embodiment, the pharmaceutical composition of the invention is free from sodium phosphate buffer.

In one embodiment, the pharmaceutical composition is explicitly free from citrate buffer. As used herein, 'explicitly free from' means that the composition does not contain any intentionally added citrate-based buffering agents, including citric acid or its salts, such as sodium citrate or potassium citrate.

In another embodiment, the pharmaceutical composition is explicitly free from carbonate buffer. As used herein, 'explicitly free from' means that the composition does not contain any intentionally added carbonate-based buffering agents, including sodium carbonate, potassium carbonate, sodium bicarbonate, or potassium bicarbonate.

In another embodiment, the pharmaceutical composition is explicitly free from borate buffer. As used herein, 'explicitly free from' means that the composition does not contain any intentionally added borate-based buffering agents, including boric acid, sodium borate, or potassium borate.

In another embodiment, the pharmaceutical composition of the invention further comprises sodium chloride and water.

In another embodiment, the pH of said pharmaceutical composition further comprising sodium chloride and water is between 6.5-8.5. In a preferred embodiment, the pH is 6.5-7.5, in an even further preferred embodiment the pH is 6.8-7.3.

In an embodiment, the pharmaceutical composition according to the invention has a total impurity content after 1 month storage at 25ºC/60%RH is below 5%, preferably below 4%, even more preferably below 3%, and most preferably below 2%.

In an embodiment, the pH of the said pharmaceutical composition after 1 month storage at 25ºC/60%RH does not suffer a variation greater than 0.5 units from the initial time point.

In an embodiment, the pharmaceutical composition is to be administered parenterally. More preferably, the route of administration is intramuscular, intravenous or subcutaneous, in a more preferrend embodiment the administration route is subcutaneous.

In one embodiment, the pharmaceutical composition is indicated for the treatment, prevention, or management of metabolic disorders and conditions associated with dysregulated glucose and energy homeostasis. Such conditions include, but are not limited to, type 2 diabetes mellitus, obesity, metabolic syndrome, insulin resistance, and cardiovascular complications related to metabolic dysfunction. In certain embodiments, the pharmaceutical composition is administered to a subject in need thereof to improve glycemic control, enhance insulin sensitivity, promote weight loss, and reduce cardiovascular risk factors.

The second aspect of the invention relates to a method for the preparation of the said above pharmaceutical composition which comprises the following steps:

   i) preparing a solution comprising the tonicity modifier, the buffering agent, the GLP-1 and GIP dual receptor agonist and water for injection; and
   ii) adjusting the pH of the final solution between 6.5-8.5, preferably between 6.5-7.5, with a pH adjuster.

In an embodiment, the solution of step i) is prepared by mixing all the inactive ingredients and the active ingredients directly into one unique solution. Alternatively, the solution of step i) is prepared by mixing a first solution comprising the inactive ingredients and a second solution comprising the active ingredient to create a final solution. Preferably, the solution of step i) is prepared by combining a first solution and a second solution to for a final solution.

In a further embodiment, the non-inorganic buffering agent is the buffering agent of the first aspect of the invention.

In a further embodiment, the dual receptor agonist is the active ingredient of the first aspect of the invention.

In a further embodiment, the final solution is prepared at a temperature between 2-15ºC, preferably 2-10ºC, more preferably 2-8 ºC, most preferably 3-6ºC. Cooling a pharmaceutical reactor to 2-15°C is generally more energy efficient than heating it to 50°C, as refrigeration systems can remove heat with a lower energy input compared to direct heating methods. Additionally, heat loss to the environment naturally aids cooling, whereas heating requires continuous energy input to maintain high temperatures.

In an embodiment, the method may comprise bubbling nitrogen gas in any of the first, second and/or final solutions, preferably until the dissolved oxygen content is < 2.0 ppm. It has been found out that bubbling nitrogen gas helps to avoid the formation of fibrils, particularly when the dissolved oxygen content is kept below 2.0 ppm.

In an embodiment, the method may comprise filtering the final solution through a 0.2 μm pore size filter. Filtering the final solution through a 0.2 μm pore size filter delays the formation of fibrils.

In an embodiment, the tonicity modifier may be selected from the group consisting of mannitol, sorbitol, lactose, propylene glycol dextrose, trehalose, sodium chloride, potassium chloride, glycerol, glycerin or mixtures thereof, preferably sodium chloride.

In an embodiment, the method comprises filling aseptically the final solution into cartridges flushed with nitrogen gas. The nitrogen gas flushed in the cartridges delays the appearance of fibrils. In an embodiment, no air bubbles remain inside the cartridges. The inventors have surprisingly found that when air bubbles remain in the cartridge, even if nitrogen is flushed, the concentration of fibrils is higher in the pharmaceutical composition according to the invention

A third aspect of the invention relates to a cartridge comprising the pharmaceutical composition of the first aspect or the pharmaceutical composition prepared according the second aspect.

Further embodiments of the invention can be found in the following numbered clauses:

1. A pharmaceutical composition comprising a GLP-1 and GIP dual receptor agonist and a non-inorganic buffer having a pKa of at least 6.6 or above.

2. The pharmaceutical composition of the preceding claim wherein the buffer is a zwitterionic buffer.

3. The pharmaceutical composition of clause 1 wherein the buffer is an organic amine-based buffer.

4. The pharmaceutical composition according to clause 2, wherein the buffer is HEPES, MOPS or PIPES, preferably the buffer is HEPES.

5. The pharmaceutical composition of clause 3, wherein the buffer is tris buffer.

6. The pharmaceutical composition according to any of the preceding clauses, wherein the dual recept agonist is tirzepatide.

7. The pharmaceutical composition according to any of the preceding clauses, wherein said composition is free from sodium phosphate buffer.

8. The pharmaceutical composition according to any of the preceding clauses, further comprising sodium chloride and water, and wherein the pH of said composition ranges between 6.5 to 7.5.

9. The pharmaceutical composition according to any of the preceding clauses, wherein the total impurity content after 1 month storage at 25$\underline{o}$C/60%RH is below 5%, preferably below 4%, even more preferablhy below 3%, and most preferably below 2%.

10. The pharmaceutical composition according to any of the preceding clauses, wherein the pH of the said composition after 1 month storage at 25$\underline{o}$C/60%RH has not suffered a variation greater than 0.5 units from the initial time point.

11. The pharmaceutical composition according to any of the preceding clauses, wherein said composition is for sub-cutaneous administration.

12. A method for the preparation of a pharmaceutical composition comprising a GLP-1 and GIP dual receptor agonist, a tonicity modifier and a non-inorganic buffering agent having a pKa of at least 6.6 or above which comprises the following steps:

preparing a solution comprising the tonicity modifier, the buffering agent, the GLP-1 and GIP dual receptor agonist and water for injection; and

adjusting the pH of the final solution between 6.5-8.5, preferably between 6.5-7.5, with a pH adjuster.

13. The method according to the preceding clause wherein the solution is prepared at a temperature between 2-15$\underline{o}$C, preferably 2-10$\underline{o}$C, more preferably 2-8 $\underline{o}$C, most preferably 3-6$\underline{o}$C.

14. The method according to the preceding clause wherein the dual receptor agonist is tirzepatide.

15. The method according to any of the three preceding clauses, wherein the buffering agent is selected from the list consisting of HEPES and tris buffer.

16. The method according to any of the four preceding clauses wherein the tonicity modifier agent is sodium chloride.

17. A method according to any of the clauses 13-16, wherein the final solution temperature does not exceed 15$\underline{o}$C.

18. A method according to any of the clauses 13-16, wherein the final solution temperature does not exceed 14$\underline{o}$C.

19. A method according to any of the clauses 13-16, wherein the final solution temperature does not exceed 13$\underline{o}$C.

20. A method according to any of the clauses 13-16, wherein the final solution temperature does not exceed 12$\underline{o}$C.

21. A method according to any of the clauses 13-16, wherein the final solution temperature does not exceed 11$\underline{o}$C.

22. A method according to any of the clauses 13-16, wherein the final solution temperature does not exceed 10$\underline{o}$C.

23. A method according to any of the clauses 13-16, wherein the final solution temperature does not exceed 9$\underline{o}$C.

24. A method according to any of the clauses 13-16, wherein the final solution temperature does not exceed 8$\underline{o}$C.

25. A cartridge comprising the pharmaceutical composition according to any of the clauses 1-11 or the compositions directly obtained by the method according to any of the clauses 12-24.

26. A pharmaceutical composition comprising tirzepatide and HEPES buffer.

27. A pharmaceutical composition according to the preceding clause further comprising sodium chloride and water, and wherein the pH of said composition ranges between 6.5 to 7.5.

28. The pharmaceutical composition according to any of the two preceding clauses wherein said composition is free from citrate buffer and free from phosphate buffer.

29. The pharmaceutical composition according to any of the three preceding clauses with the proviso that the composition is not a hydrogel composition.

30. The pharmaceutical composition according to any of the four preceding clauses wherein the composition is a solution for sub-cutaneous administration.

31. The pharmaceutical composition according to any of the five preceding clauses wherein said composition is free from hydrogel forming polymers.

32. A pharmaceutical composition comprising a tirzepatide and tris buffer.

33. A pharmaceutical composition according to the preceding clause further comprising sodium chloride and water, and wherein the pH of said composition ranges between 6.5 to 7.5.

34. The pharmaceutical composition according to any of the two preceding clauses wherein said composition is free from citrate buffer and free from phosphate buffer.

35. The pharmaceutical composition according to any of the three preceding clauses with the proviso that the composition is not a hydrogel composition.

36. The pharmaceutical composition according to any of the four preceding clauses wherein the composition is a solution for sub-cutaneous administration.

37. The pharmaceutical composition according to any of the five preceding clauses wherein said composition is free from hydrogel forming polymers.

38. A pharmaceutical composition according to any of the clauses 1-11 with the proviso that the composition is not a hydrogel composition.

39. A pharmaceutical composition according to the preceding clause wherein the composition is a solution for sub-cutaneous administration.

40. A pharmaceutical composition according to any of the two preceding clauses wherein the composition is free from hydrogel forming polymers.

41. A pharmaceutical composition according to any of the clauses 1-11 or 38-40 wherein the dual receptor agonist concentration is 5 mg/mL.

42. A pharmaceutical composition according to any of the clauses 1-11 or 38-41 comprising sodium chloride as tonicity agent at a molar concentration of 0.14M.

43. A pharmaceutical composition according to any of the clauses 1-11 or 38-42 wherein the oxygen content is < 2.0 ppm.

## EXAMPLES

### Example 1. Preparation of pharmaceutical compositions comprising tirzepatide

| Component | GLN-1 | GLN-2 | Comp. example 1 |
|---|---|---|---|
| Tirzepatide | 5 | 5 | 5 |
| Sodium Chloride | 8.2 | 8.2 | 8.2 |
| Sodium Phosphate dibasic heptahydrate | - | - | 1.4 |
| Tris buffer | - | 0.63 | - |
| HEPES buffer | 1.19 | - | - |
| HCl/NaOH | q.s. to pH 7 | q.s. to pH 7 | q.s. to pH 7 |
| Water for injection | q.s. to 1mL | q.s. to 1mL | q.s. to 1mL |
| Nitrogen | q.s | q.s | q.s |

The amount of the different components is expressed as mg/mL.

The GLN-1, GLN-2 and Comp. Example 1 formulations were manufactured by preparing a first solution comprising sodium chloride, the corresponding buffering agent, and water for injection. In another container, a second solution comprising tirzepatide and water for injection was prepared. Then, a final solution was prepared by mixing the said first and the said second solution. The first, second and final solutions were prepared at a temperature between 2-8 $\underline{o}$C. Nitrogen gas was bubbled in the first, second and final solution, to maintain the dissolved oxygen content below 2.0 ppm. Then, the pH of the final solution was adjusted to 7 with HCl and/or NaOH. Eventually, the final solution was filtered through a 0.2 $\mu$m filter.

## Example 2. Impurity content

All formulations obtained in Example 1 were analysed by UPLC as described above for impurity content at two different times, freshly prepared (t=0) and at 1 month stored at 25 ºC / 60 % RH. The following results were obtained:

|  | GLN-1 | | GLN-2 | | Comp. Ex. 1 | |
|---|---|---|---|---|---|---|
|  | t=0 | t=1 | t=0 | t=1 | t=0 | t=1 |
| TZP-L-beta-Asp15 [Impurity A] ($t_{Rr}$ = 0.86) | ND | 0.05 | ND | 0.03 | ND | ND |
| Unknown Impurity ($t_{Rr}$ = 0.97) | 0.04 | 0.03 | 0.03 | 0.01 | 0.03 | 0.01 |
| D-Ser32+33 [Impurity C] ($t_{Rr}$ = 1.03) | 0.02 | 0.05 | 0.02 | 0.05 | 0.02 | 0.04 |
| TZP-L-beta-Asp9 [Impurity D] ($t_{Rr}$ = 1.05) | 0.04 | 0.15 | 0.04 | 0.14 | 0.05 | 0.14 |
| Unknown Impurity ($t_{Rr}$ = 0.32) | ND | 0.02 | ND | 0.02 | ND | 0.43 |
| Unknown Impurity ($t_{Rr}$ = 0.88) | ND | 0.06 | ND | 0.12 | ND | ND |
| Unknown Impurity ($t_{Rr}$ = 0.90) | ND | 0.80 | ND | 1.08 | ND | 1.90 |
| Unknown Impurity ($t_{Rr}$ = 0.92) | ND | 0.05 | ND | ND | ND | ND |
| Unknown Impurity ($t_{Rr}$ = 0.95) | 0.02 | 0.03 | 0.03 | 0.02 | 0.03 | 0.06 |
| Total impurities (%) | 0.12 | 1.24 | 0.12 | 1.47 | 0.13 | 2.58 |

All amounts of impurities are expressed as a percentage, "ND" corresponds to not detected. As it can be seen, the amount of the majority of the impurities over time is lower in GLN-1 and GLN-2, as well as the percentage of total impurities.

## Example 3. Thioflavin T (ThT) Fibrillation Estimation Assay

Low physical stability of a peptide may lead to amyloid fibril formation, which is observed as well-ordered, thread-like macromolecular structures in the sample eventually resulting in gel formation. This has traditionally been measured by visual inspection of the sample. However, that kind of measurement is very subjective and depends on the observer. Therefore, the application of a small molecule indicator test is much more advantageous. Thioflavin T (ThT) is such a test and has a distinct fluorescence signature when binding to fibrils.

To minimise costs related to the API and being able to estimate the fibrils in the compositions (at 5 mg/mL) by ThT fibrillation assay, Bovine serum albumin (BSA) was used for the standard curve of the assay, since this protein has similar tendency to fibril formation as tirzepatide. To this aim, a BSA standard curve was prepared from 0.188 to 0.003 mM and a Thioflavin-T working solution at 50 uM. After preparation of standard concentration and one blank (PBS), the BSA standard samples at 80ºC for 30 min and then cool at room temperature for fibril generation.

Preparation of the samples

Preparation of standard Blank: Add 50μL of heated and cooled PBS into 96 well plate in duplicate.

Preparation of Standard: Add 50 μL of heated and cooled BSA (0.188 mM - 0.003 mM) each into 96 well plate in duplicate.

Preparation of Test samples Blank: Add 50μL of Milli-Q water into 96 well plate in duplicate.

Preparation of Test samples: Add 50 μL Test samples (50μM) into 96 well plate in duplicate. Then read the plate as per method created in Micro plate reader.

After addition of all blanks, standard curve concentrations and test sample concentrations in the plate, added 150μL of thioflavin-T working solution(50μM) into each well and mixed gently by pipetting. Read the plate as per the following microplate reader conditions:

(Read Mode: Fluorescence; excitation wavelength: 440nm; emission wavelength: 482nm; bandwidth excitation: 9 nm; bandwidth emission: 15 nm; plate type: 96 well standard opaque; plate height: 14.6mm; plate Shake: 60 seconds, orbital, medium (before first read); PMT and optics: 6 flashes/read; Read from: Top; Read height: 1.00mm)

Calculations and results

Plotted the standard curve with the blank subtracted values of standards on Y-axis and their concentrations on X-axis.

Derived slope (Mstd), intercept (Cstd) and regression(R2) from the straight-line equation.

The following compositions were measured according to the above ThT fibrillation assay:

| Composition | Temperature (ºC) | pH |
|---|---|---|
| GLN-1 | 8 | 7.0 |
| GLN-2 | 8 | 7.0 |
| Comp. Ex. 1 | 8 | 7.0 |
| Comp. Ex. 2 | 60 | 7.0 |

GLN-1, GLN-2 and Comp. Ex. 1 were prepared as described in Example 1. Comp. Ex. 2 was prepared as Comp. Ex. 1 but the process was carried by heating the solutions during the whole process at 60ºC.

After preparation, the samples were stored for 1 month at 30ºC / Relative Humidity 65% and analysed. Afterwards, samples were analysed according to the ThT fibrillation assay detailed before and the following results were obtained:

| Composition | Fibrils content (μM) |
|---|---|
| GLN-1 | 0,0255 |
| GLN-2 | 0,0392 |
| Comp. Ex. 1 | 0,0282 |
| Comp. Ex. 2 | 0,1459 |

## Claims

1. A pharmaceutical composition comprising a GLP-1 and GIP dual receptor agonist and a non-inorganic buffer having a pKa of at least 6.6 or above.

2. The pharmaceutical composition of the preceding claim wherein the non-inorganic buffer is a zwitterionic buffer.

3. The pharmaceutical composition of claim 1 wherein the non-inorganic buffer is an organic amine-based buffer.

4. The pharmaceutical composition according to claim 2, wherein the zwitterionic buffer is HEPES, MOPS or PIPES preferably, the buffer is HEPES.

5. The pharmaceutical composition of claim 3, wherein the organic amine-based buffer is tris buffer.

6. The pharmaceutical composition according to any of the preceding claims, wherein the dual recept agonist is tirzepatide.

7. The pharmaceutical composition according to any of the preceding claims, wherein said composition is free from sodium phosphate buffer.

8. The pharmaceutical composition according to any of the preceding claims, further comprising sodium chloride and water, and wherein the pH of said composition ranges between 6.5 to 7.5.

9. The pharmaceutical composition according to any of the preceding claims, wherein the total impurity content after 1 month storage at 25ºC/60%RH is below 5%, preferably below 4, even more preferablhy below 3%, and most preferably below 2%.

10. The pharmaceutical composition according to any of the preceding claims, wherein the pH of the said composition after 1 month storage at 25ºC/60%RH has not suffered a variation greater than 0.5 units from the initial time point.

11. The pharmaceutical composition according to any of the preceding claims, wherein said composition is for subcutaneous administration.

12. A method for the preparation of the pharmaceutical composition of claim 1 which comprises the following steps:

   i) preparing a solution comprising the tonicity modifier, the buffering agent, the GLP-1 and GIP dual receptor agonist and water for injection; and
   ii) adjusting the pH of the final solution between 6.5-8.5, preferably between 6.5-7.5, with a pH adjuster.

13. The method according to the preceding claim wherein the dual receptor agonist is tirzepatide.

14. The method according to any of the two preceding claims, wherein the buffering agent is selected from the list consisting of HEPES and tris buffer.

15. A cartridge comprising the pharmaceutical composition according to any of the claims 1-11 or the compositions directly obtained by the method according to any of the claims 12-14.

Europäisches
Patentamt
European
Patent Office
Office européen
des brevets

# EUROPEAN SEARCH REPORT

**Application Number**

EP 25 38 2104

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2024/006662 A1 (LILLY CO ELI [US]) 4 January 2024 (2024-01-04) * the whole document * * claims 1-98 * ----- | 1-15 | INV. A61K9/00 A61K9/08 A61K9/19 A61K38/26 |
| Y | WO 2020/127476 A1 (KRKA D D NOVO MESTO [SI]) 25 June 2020 (2020-06-25) * the whole document * * claims 1-14 * ----- | 1-15 | ADD. A61K47/02 A61K47/18 A61K47/26 |

**TECHNICAL FIELDS SEARCHED (IPC)**

A61K

The present search report has been drawn up for all claims

| Place of search | Date of completion of the search | Examiner |
|---|---|---|
| Munich | 1 July 2025 | Felder, Christian |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 38 2104

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2025

| Patent document cited in search report | Publication date | Patent family member(s) | | Publication date |
|---|---|---|---|---|
| WO 2024006662 A1 | 04-01-2024 | AR | 129796 A1 | 25-09-2024 |
| | | AU | 2023300973 A1 | 02-01-2025 |
| | | CL | 2024003931 A1 | 21-03-2025 |
| | | CN | 119421702 A | 11-02-2025 |
| | | CO | 2024017342 A2 | 30-12-2024 |
| | | CR | 20240551 A | 27-01-2025 |
| | | DO | P2024000269 A | 28-02-2025 |
| | | EP | 4547218 A1 | 07-05-2025 |
| | | IL | 317571 A | 01-02-2025 |
| | | KR | 20250029908 A | 05-03-2025 |
| | | MA | 71323 A | 30-04-2025 |
| | | PE | 20250835 A1 | 21-03-2025 |
| | | TW | 202417035 A | 01-05-2024 |
| | | WO | 2024006662 A1 | 04-01-2024 |
| WO 2020127476 A1 | 25-06-2020 | EP | 3897570 A1 | 27-10-2021 |
| | | WO | 2020127476 A1 | 25-06-2020 |

EPO FORM P0459

For more details about this annex : see Official Journal of the European Patent Office, No. 12/82

**REFERENCES CITED IN THE DESCRIPTION**

**Patent documents cited in the description**

- EP 3810201 A
- WO 2024006662 A
- WO 2006051110 A2
- WO 2020127476 A1
- WO 202061310 A